# FASCICULE DE BREVET EUROPEEN

(11) **EP 0 550 307 B1**
(45) Date de publication et mention de la délivrance du brevet: **26.07.1995**
(21) Numéro de dépôt: 92403438.2
(22) Date de dépôt: 17.12.1992
(51) Int. Cl.: C07C 209/26, C07C 209/48

(54) **Procédé d'obtention d'amines tertiaires méthylées par réaction d'hexaméthylènetétramine sur les amines ou les nitriles**
Verfahren zur Herstellung von methylierten tertiären Aminen durch Reaktion von Hexamethylentetramin auf Amine oder Nitrile
Process for the preparation of methylated tertiary amines by reaction of hexamethylenetetramine with amines or nitriles

(30) Priorité: 30.12.1991 FR 9116316
(43) Date de publication de la demande: 07.07.1993
(73) Titulaire: CECA S.A., 92800 Puteaux (FR)
(72) Inventeur: Fouquay, Stéphane, F-76130 Mont Saint-Aignan (FR)
(74) Mandataire: Haicour, Philippe

(56) Documents cités:
- DE-A- 2 545 695
- DE-C- 946 622
- GB-A- 860 922
- ORGANIC REACTIONS vol. 8, 1967, pages 197 - 217 S.J. ANGYAL 'The Sommelet Reaction'

## Description

La présente invention est relative à un procédé d'obtention d'amines tertiaires méthylées, qui consiste à faire réagir de l'hexaméthylène-tétramine sur un précurseur azoté en présence d'hydrogène et d'un catalyseur.

Les amines tertiaires monométhylées ou diméthylées auxquelles on peut accéder grâce à l'invention appartiennent à un ensemble très vaste d'amines cycliques ou acycliques représentables par des formules très générales de molécules diversement substituées sur l'azote, telles que 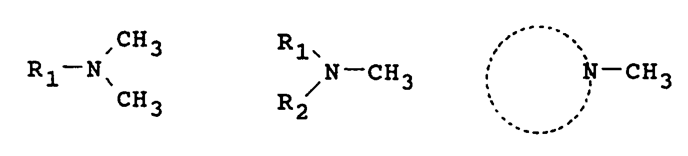
englobant entre autres des polyamines et des amines et polyamines mixtes.

Les amines tertiaires mono ou diméthylées constituent la base d'au moins trois types de dérivés très recherchés dans l'industrie: les sels d'ammonium quaternaire, les bétaïnes et les oxydes d'amines dont les applications sont nombreuses en détergence, en cosmétique, comme bactéricides, antibiotiques, comme agents de traitement des textiles, ou encore pour la fabrication des argiles organophiles.

Il existe de nombreux procédés d'obtention d'amines tertiaires méthylées, parmi lesquels on relève la méthylation directe des amines primaires ou secondaires par divers réactifs de méthylation, dont le chlorure ou le sulfate de méthyle, ou le système formol/acide formique (réaction de Leuckart-Wallach - pour références, voir Merck Index, 10^{e} édition, p.ONR-55), l'hydrogénation des dialkylamides (par exemple EP 412359 - Hoechst, ou FR 2633927 - CECA S.A.), ou encore par la transformation des nitriles ou des amines par le système formol hydrogène (JP 63287752 - Kao Soap; DE 2545695-BASF AG).

Ces procédés présentent divers inconvénients majeurs. Ainsi, ceux qui qui exploitent la réaction de Leuckart, déjà de conduite difficile, produisent des amines en outre contaminées par du formol; d'autres qui mettent en oeuvre la méthylation par le chlorure de méthyle, outre qu'ils sont générateurs d'acide chlorhydrique, présentent des défauts de sélectivité, les produits résultants contenant des proportions difficilement contrôlables de sels d'ammonium quaternaire. L'hydrogénation directe des amides procède sur catalyseur au chromite de cuivre, incompatible avec les catalyseurs au nickel utilisés par ailleurs dans les installations produisant des amines: ceci multiplie le nombre des unités et augmente les coût globaux de production de ces installations.

La demanderesse a maintenant trouvé que, dans les conditions que l'on va préciser, l'hexaméthylène-tétramine pouvait constituer un réactif précieux de méthylation des amines ou des nitriles dont les avantages apparaîtront clairement dans la présente description.

L'hexaméthylènetétramine (ci-après HMTA) est le composé bien connu, de formule générale C₆H₁₂N₄, répertorié sous le "registry number" [RN = 100-97-0], résultat de la condensation de formol et d'ammoniac. L'HMTA est un composé tétracyclique, comme l'indique son nom officiel de 1,3,5,7-tétraazatricyclo [3.3.1.1^{3,7}] décane ou, pour rappeler sa structure dans l'espace, son appellation de 1,3,5,7-tétraazaadamantane. L'HMTA est surtout utilisée, outre comme antiseptique, notamment en thérapeutique urinaire (urotropine), comme donneur de formol (durcisseur de résines formophénoliques, agent de vulcanisation des caoutchoucs, inhibiteur de corrosion du fer. C'est également une base susceptible de donner des sels cristallisés avec les acides minéraux ou organiques. L'HMTA se comporte aussi comme une amine tertiaire vis à vis des agents de quaternisation et fournit des sels d'ammonium quaternaires dont l'hydrolyse libère l'amine correspondante, ce par quoi l'HMTA fonctionne comme agent d'amination.(voir par exemple, K.E. Schulte et M. Goes, Arch. Pharm 290, 118-30, 1957).

Les réactions générales qui basent la présente invention sont représentables par l'une ou l'autre des équations ci-dessous

R₁NH₂ + x C₆H₁₂N₄ + y H₂ å R₁N(CH₃)₂ + p NH₃ + q N(CH₃)₃

ou

R₁R₂NH + x' C₆H₁₂N₄ + y' H₂ å R₁R₂NCH₃ + p' NH₃ + q' N(CH₃)₃

ou encore

R₅CN + x'' C₆H₁₂N₄ + y'' H₂ å R₅CH₂N(CH₃)₂ + p'' NH₃ + q'' N(CH₃)₃,

formules dans lesquelles les divers coefficients yⁱ, pⁱ et qⁱ dépendent et peuvent s'exprimer en fonction des valeurs respectives xⁱ. Si l'on se place, pour simplifier, dans le cas de la méthylation d'une amine primaire, la réaction optimale est représentable globalement par

R₁NH₂ + 1/3 C₆H₁₂N₄ + 2 H₂ å R₁N(CH₃)₂ + 4/3 NH₃

En pratique, la réaction s'exploite avec des quantités supérieures d'HMTA, surtout lorsqu'elle est appliquée à des précurseurs à longue chaîne grasse. Le taux de conversion du précurseur azoté est gouverné par le rapport molaire x de l'HMTA à l'azote méthylable. Ce rapport est lié à l'encombrement stérique du site azoté, donc à la structure même des précurseurs. Il impose donc la quantité d'hydrogène consommé y, et la composition en sous-produits de réaction, NH₃ et triméthylamine, dans les effluents gazeux.

La méthylation par l'HMTA est une réaction sélective. On entend par là que dans les conditions qui sont celles de l'invention, seules les fonctions amines ou nitriles participent à la réaction. Il est ainsi possible d'utiliser comme précurseurs azotés des produits aussi divers que les aminoalcools, les amidoamines ou les étheramines en respectant l'intégrité des fonctions associées à la fonction amine ou nitrile de ces précurseurs. La réaction permet ainsi d'atteindre des méthylamines très variées en s'adressant à de très nombreux précurseurs azotés.
Le procédé selon l'invention permet ainsi l'obtention d'alkyldiméthylamines grasses à partir de précurseurs azotés comportant une fonction amine primaire, molécules représentables par une formule générale R₁NH₂: monoamines vraies, où R₁ est un reste R hydrocarboné linéaire ou ramifié, saturé ou insaturé comportant de 10 à 22 atomes de carbone; aminoalcools, avec R₁ constitué d'un reste monovalent tel HO-CH₂-CH₂- ou HO-CH(CH₃)-CH₂-; étheramines, dans lesquelles R₁ est un groupe R-O-(CH₂)₃-, R ayant la même définition que ci-dessus; étheramines, R₁ y ayant la signification R-(OCH₂-CH₂)ₚ, R-[OCH(CH₃)-CH₂]ₚ ou R-[OCH₂-CH(CH₃)]ₚ, dans lesquels R a la même signification que ci-dessus et où p est un nombre qui peut prendre toute valeur statistique positive.

Les précurseurs azotés utilisables dans la réaction selon l'invention sont aussi les amines secondaires, de formule générale R₁NHR₂ simples ou complexes, où R₁ a la même définition que précédemment, où R₂ peut être CH₃ ou R₁, R₁ et R₂ pouvant en outre être engagés dans un cycle, en l'occurence amine cyclique comportant le cas échéant un second hétéroatome azote ou oxygène.

La méthylation par l'HMTA est évidemment applicable aux polyamines, à commencer par les diamines α-ω, H₂N-(CH₂)ᵣ-NH₂ où r est un nombre entier pouvant prendre toute valeur entière égale ou supérieure à 2 et qui peut être très grande, bien que pratiquement on ne dépasse pas les valeurs de 14/16, et d'une façon générale les polyamines représentables par une formule telle que 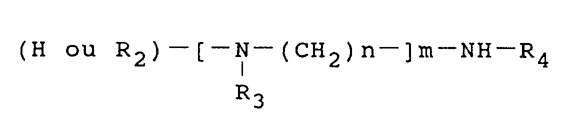
où R₁ a la même définition que précédemment,
où R₂ a la signification précédente de CH₃ ou R₁,
où R₃ est H ou CH₃ ou R₁,
où R₄ est H ou CH₃ ou R ou R₁,
où n est un entier pouvant prendre les valeurs 2 ou 3,
où m est un entier ou un nombre quelconque à valeur statistique supérieure à 1,
R₂ et R₃ pouvant en outre être refermés pour former une amine cyclique ou même un cycle azoté contenant un autre hétéroatome azoté ou oxygéné.

Ces polyamines comprennent en particulier les diamines, comme les alkylpropylènes diamines, que le procédé selon l'invention transforme en des mélanges plus ou moins complexes de polyamines méthylées telles que 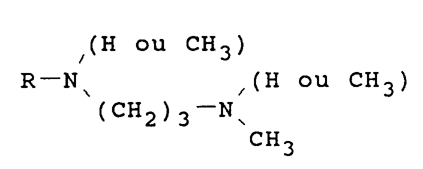
Les dérivés correspondants à chaîne grasse, et plus précisément à chaîne suif sont industriellement très intéressants. En effet, les alkylpropylène-diamines grasses à base suif sont des produits très utilisés par l'industrie routière comme émulsifiants des bitumes. Elles ont l'inconvénient d'être solides ou pâteuses: les diamines méthylées qu'on obtient en leur appliquant le procédé selon l'invention en ont conservé les remarquables propriétés émulsifiantes, mais ont des points de fusion plus bas, ce qui en facilite très notablement la manipulation et le dosage. Elles constituent de plus les précurseurs des chlorures d'alkylpropylènes di-ammoniums quaternaires également utilisés dans l'industrie routière.

Parmi les nombreuses autres polyamines utilisables comme précurseurs azotés pour le procédé selon l'invention, il convient également de préciser les polyamines grasses, linéaires ou ramifiées sur l'azote selon l'une ou l'autre des formules générales

R―NH―[(CH₂)n ―NH]m ―(CH₂)n ―NH₂

ou 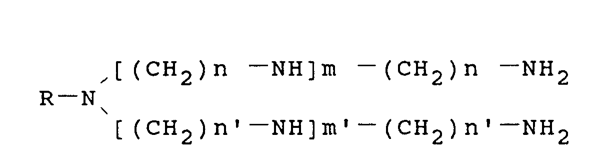
où R est une chaîne hydrocarbonée comportant de 10 à 22 atomes de carbone;
où n et n' sont des entiers pouvant prendre les valeurs 2 ou 3;
où m et m' peuvent prendre toute valeur entière ou statistique de O à 3.

Ces produits, et notamment les polyamines à chaîne suif représentables par la première de ces formules, et dans laquelle n est 3 et m une valeur statistique d'environ 2 à 3, sont également utilisés dans l'industrie routière comme dopes d'adhésivité et émulsifiants des bitumes; la méthylation selon l'invention les transforme en des produits à point de fusion contrôlé, lequel dépend du taux d'alkylation.

Les amidoamines ou amidopolyamines grasses, produits industriels souvent en compétition avec les précédents, représentables par

R-CO-[NH-(CH₂)ₙ-]ₘ-NH-(CO-R ou H)

où R est un reste hydrocarboné comme précédemment défini, où n a généralement la valeur 2 ou 3, et m une valeur statistique de 1 à 6, ainsi que leurs dérivés plus ou moins cyclisés en imidazolines ou tétrahydropyrimidines, sont également méthylables par le procédé selon l'invention.

Le procédé selon l'invention permet également l'accès aux amines méthylées directement à partir des nitriles, soit à partir des mononitriles gras, simples ou complexes tels que

R₅―C≡N

où R₅ est R ou R-O-(CH₂)₂-,
mais aussi à partir d'aminonitriles 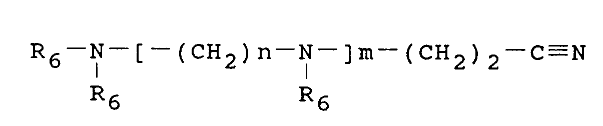
formule dans laquelle les R₆ peuvent être indépendamment les uns des autres H ou CH₃ ou R₁, deux R₆ lorsqu'ils sont substituants sur le même azote pouvant en outre être bouclés formant une amine cyclique, laquelle pouvant même contenir un autre hétéroatome azoté ou oxygéné, et où n est un entier pouvant prendre les valeurs 2 ou 3, m est un entier ou un nombre à valeur statistique pouvant prendre toute valeur de 0 à 3. La sélectivité de la réaction qui a été signalée plus haut permet la méthylation à la fois des azotes amines et nitriles de ces précurseurs, tout en respectant les autres fonctions éventuellement associées.

Parmi ces produits, mention doit être faite en particulier des dinitriles

N≡C―(CH₂)q―C≡N

où q est un entier pouvant prendre toute valeur égale ou supérieure à 2, valeurs ne dépassant cependant pas 14 dans la pratique, et les aminonitriles intermédiaires de la synthèse industrielle des polyamines par cyanoéthylation, et qu'on représente par l'une ou l'autre des formules

R₆―NH―[―(CH₂)n―NH―]m―(CH₂)₂―C≡N

ou 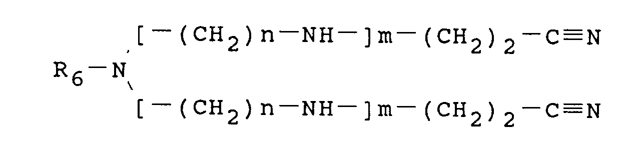
où R₆ est H ou CH₃ ou R₁, où n est un entier pouvant prendre les valeurs 2 ou 3, et où m est un entier pouvant prendre toute valeur entière ou statistique de 0 à 3.

D'une façon générale, la préparation d'amines tertiaires méthylées selon l'invention par réaction de précurseurs azotés et d'HMTA est conduite sur catalyseurs au nickel supporté, sous des pressions d'hydrogène comprises entre 3 et 50 bars, et à des températures au plus égales à 160°C.

Le procédé selon l'invention présente l'avantage de ne pas donner lieu à formation de mousses, comme celles qui gênent considérablement le déroulement des procédés qui exploitent la réaction de Leuckart. Les réactions mises en jeu engendrent les amines tertiaires visées avec des sous-produits limités, tant qualitativement que quantitativement; il n'est donc pas nécessaire de procéder à des lavages répétés pour éliminer les impuretés des produits finals. C'est probablement pour cette raison que les dérivés quaternaires (sels d'ammonium quaternaire ou bétaïnes) formés à partir des méthylalkylamines obtenues selon l'invention ne présentent pas les colorations roses qui affectent ces produits lorsqu'ils sont issus des amines tertiaires obtenues selon les procédés de l'art antérieur.

Les exemples suivants feront bien comprendre l'invention.

### Exemple 1 : méthylation de monoamine de coprah.

Dans un réacteur de 2 litres, on charge 700 grammes d'une monoalkylamine commerciale (Noram^C de CECA S.A., dont la répartition des chaînes est celle des acides de coprah dont elle est issue, c'est à dire riche en chaînes à 12-14 carbones, d'alcalinité égale à 5,20; ce produit contient environ 3,8% en poids d'amines secondaires dialkyles), 363 grammes d'HMTA (rapport molaire amine/HMTA = 0,70) et 7 grammes d'un catalyseur au nickel supporté du type E 230 P (Degussa) à 63% de nickel (soit 1% de nickel par rapport à la monoalkylamine).

Sous agitation à 1100/1500 tr/min, on dégaze l'ammoniac et la triméthylamine formés par étape à 130°C lorsque la consommation d'hydrogène est nulle, puis on remonte à 155°C et ainsi de suite jusqu'à absence totale de consommation à 155°C. La pression d'hydrogène est de 20 bars. L'opération dure de 5 à 7 heures.

Après séparation du catalyseur, l'amine brute récupérée a la composition suivante:

| Composition | % en poids |
|---|---|
| Monoalkylamines | 0,81 |
| Monométhylmonoalkylamines R-NH-CH₃ | 3,7 |
| Diméthylmonoalkylamines R-N-(CH₃)₂ | 89,1 |
| Dialkylamines R-NH-R | 3,4 |
| Monométhyldialkylamines (R-NCH₃-R) | 3,0 |

On peut aisément en séparer par simple distillation la composante lourde dialkyle, laquelle provient pour une part non négligeable des dialkylamines présentes dans l'amine de départ. On obtient alors un mélange dont la composition s'établit à

| Composition | % en poids |
|---|---|
| Monoalkylamines | 0,9 |
| Monométhylmonoalkylamines R-NH-CH₃ | 3,9 |
| Diméthylmonoalkylamines R-N-(CH₃)₂ | 95,2 |

### Exemple 2: préparation de diméthylalkylamine à partir d'un nitrile.

Dans un réacteur identique à celui de l'exemple 1, on charge 700 g d'un nitrile à base coprah, 1,6% du même catalyseur E 230 P que précédemment, et 371 g d'HMTA (rapport molaire de 0,70). La réaction est conduite dans les mêmes conditions que dans l'exemple 1.

On obtient:

| Composition | % en poids |
|---|---|
| Monoalkylamines | négligeable |
| Monométhylmonoalkylamines R-NH-CH₃ | négligeable |
| Diméthylmonoalkylamines R-N-(CH₃)₂ | 76,8 |
| Dialkylamines R-NH-R | 4,5 |
| Monométhyldialkylamines (R-NCH₃-R) | 19,7 |

### Exemple 3: préparation de N-méthyl-dialkylamine de suifhydrogéné à partir de la dialkylamine correspondante.

Dans un réacteur identique à celui de l'exemple 1, on charge 700 g d'une dialkylamine de suif hydrogéné (Noram^ 2SH de CECA S.A.), 1,6% en poids du même catalyseur E 230 P que précédemment, et 72,3 g d'HMTA (rapport molaire amine/HMTA = 1/0,37). Après réaction, on obtient:

| Composition | % en poids |
|---|---|
| Monoalkylamines | négligeable |
| Monométhylmonoalkylamines R-NH-CH₃ | négligeable |
| Diméthylmonoalkylamines R-N-(CH₃)₂ | négligeable |
| Dialkylamines R-NH-R | 56,0 |
| Monométhyldialkylamines (R-NCH₃-R) | 44,0 |

Avec un chargement de 136 g d'HMTA, (rapport molaire 1/0,70), toutes les autres caractéristiques étant inchangées, on obtient:

| Composition | % en poids |
|---|---|
| Monoalkylamines | négligeable |
| Monométhylmonoalkylamines R-NH-CH₃ | négligeable |
| Diméthylmonoalkylamines R-N-(CH₃)₂ | négligeable |
| Dialkylamines R-NH-R | 28,4 |
| Monométhyldialkylamines (R-NCH₃-R) | 71,6 |

La réaction de l'HMTA sur les dialkylamines grasses est particulièrement intéressante en ce qu'il n'y a pas de sous-produit de réaction et que le produit final que l'on recueille est la méthyldialkylamine avec la dialkylamine de départ.

### Exemple 4: réaction de la diéthanolamine et de l'HMTA.

Dans l'appareillage utilisé dans l'exemple 1, on charge 700 g de diéthanolamine et 346 g d'HMTA (rapport molaire = 1/0,35). Après réaction dans les conditions de l'exemple 1, on obtient :

| Composition | % en poids |
|---|---|
| Diéthanolamine (HOCH₂CH₂)₂NH | 8,1 |
| Méthyl-diéthanolamine (HOCH₂CH₂)₂NCH₃ | 91,9 |

Le produit de cette réaction est un produit simple, mélange du seul produit de réaction, la méthyldiéthanolamine avec la diéthanolamine de départ.

La méthyldiéthanolamine est un intermédiaire fort intéressant pour l'obtention d'amino-esters et de leurs dérivés d'ammonium quaternaire, utilisables, notamment pour la formulation d'adoucissants textiles biodégradables.

L'exemple est transposable directement à la méthylation de la monoéthanolamine, pour donner essentiellement de la monométhyléthanolamine et de la diméthyléthanolamine.

## Revendications

1. Procédé pour l'obtention d'amines tertiaires méthylées caractérisé en ce que l'on fait réagir l'hexaméthylène-tétramine sur des précurseurs azotés pris dans le groupe constitué par les amines primaires ou secondaires, les nitriles et les aminonitriles, la réaction étant conduite sous pression d'hydrogène et en présence d'un catalyseur d'hydrogénation, à une température au plus égale à 160°C.

2. Procédé selon la revendication 1 dans lequel le catalyseur est un catalyseur d'hydrogénation au nickel supporté.

3. Procédé selon les revendications 1 ou 2, caractérisé en ce que le précurseur azoté est une monoamine primaire R₁NH₂, où R₁ est:
- un reste R, hydrocarboné linéaire ou ramifié, saturé ou insaturé comportant de 10 à 22 atomes de carbone, ou
- un groupe HO-CH₂-CH₂- ou HO-CH(CH₃)-CH₂-, ou
- un groupe R-O-(CH₂)₃-, dans lequel R est un reste hydrocarboné linéaire ou ramifié, saturé ou insaturé comportant de 10 à 20 atomes de carbone, ou
- un groupe R-(OCH₂-CH₂)ₚ, R-[OCH(CH₃)-CH₂]ₚ ou R-[OCH₂-CH(CH₃)]ₚ, dans lequel R a la même signification que ci-dessus et où p est un nombre qui peut prendre toute valeur statistique positive.

4. Procédé selon les revendications 1 ou 2, caractérisé en ce que le précurseur azoté est une monoamine secondaire R₁NHR₂,
où R₁ a la même définition que dans le revendication 3,
où R₂ peut être CH₃ ou R₁,
R₁ et R₂ pouvant être refermés en amine cyclique, le cycle pouvant contenir accessoirement un autre hétéroatome azoté ou oxygéné.

5. Procédé selon la revendication 4, caractérisé en ce que le précurseur azoté est la monoéthanolamine ou la diéthanolamine.

6. Procédé selon les revendications 1 ou 2, caractérisé en ce que le précurseur azoté est une diamine primaire H₂N-(CH₂)ᵣ-NH₂ où r est un nombre entier pouvant prendre toute valeur égale ou supérieure à 2, généralement comprise entre 2 et 16.

7. Procédé selon les revendications 1 ou 2, caractérisé en ce que le précurseur azoté est une polyamine représentable par la formule générale 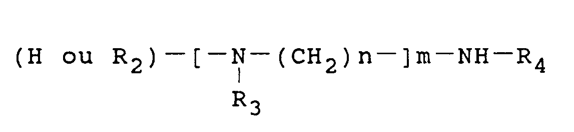 où R₁ a la même définition que dans le revendication 3,
où R₂ peut être CH₃ ou R₁,
où R₃ peut être H ou CH₃ ou R₁
où R₄ est H ou CH₃ ou R ou R₁,
où n est un entier pouvant prendre les valeurs 2 ou 3,
où m est un entier ou un nombre quelconque à valeur statistique supérieure à 1,
R₂ et R₃ pouvant en outre être refermés pour former une amine cyclique ou même un cycle azoté contenant un autre hétéroatome azoté ou oxygéné.

8. Procédé selon les revendications 1 ou 2, caractérisé en ce que le précurseur azoté est une polyamine grasse répondant à l'une ou l'autre des formule
R―NH―[(CH₂)n ―NH]m ―(CH₂)n ―NH₂
ou 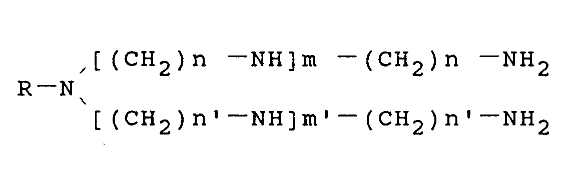 où R est une chaîne hydrocarbonée comportant de 10 à 22 atomes de carbone;
où n et n' sont des entiers pouvant prendre les valeurs 2 ou 3;
où m et m' peuvent prendre toute valeur entière ou statistique de O à 3.

9. Procédé selon la revendication 8, caractérisé en ce que le précurseur azoté est une diamine grasse répondant à la formule générale S-NH-(CH₂)₃-NH₂, S étant une chaîne hydrocarbonée à base suif.

10. Procédé selon la revendication 8, caractérisé en ce que le précurseur azoté est une polyamine grasse à chaîne suif, de formule RNH-[(CH₂)₃-NH]ₘ-(CH₂)₃-NH₂, où m a une valeur statistique d'environ 2 à 3.

11. Procédé selon les revendications 1 ou 2, caractérisé en ce que les précurseurs azotés sont des amidoamines ou amidopolyamines grasses représentables par R-CO-[NH-(CH₂)ₙ-]ₘ-NH-(CO-R ou H), où R est un reste hydrocarboné défini comme précédemment, où n a généralement la valeur 2 ou 3, et m une valeur statistique de 1 à 6, ainsi que leurs dérivés plus ou moins cyclisés en imidazolines ou tétrahydropyrimidines

12. Procédé selon les revendications 1 ou 2, caractérisé en ce que le précurseur azoté est un mononitrile représentable par la formule générale
R₅―C≡N
où R₅ est R ou R-O-(CH₂)₂ -, R ayant la définition donnée plus haut.

13. Procédé selon les revendications 1 ou 2, caractérisé en ce que le précurseur azoté est un aminonitrile représentable par la formule générale 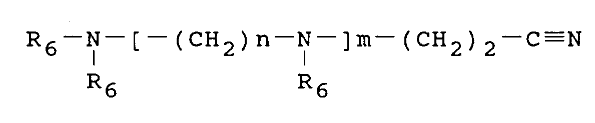 dans laquelle
- les R₆ peuvent être indépendamment les uns des autres H ou CH₃ ou R₁,
deux R₆ lorsqu'ils sont substituants sur le même azote pouvant en outre constituer un cycle avec un hétéroatome azoté ou oxygéné,
et où
- n est un entier pouvant prendre les valeurs 2 ou 3,
- m peut prendre toute valeur de O à 3.

14. Procédé selon les revendications 1 ou 2, caractérisé en ce que le précurseur azoté est un dinitrile
N≡C―(CH₂)q―C≡N
où q est un entier pouvant prendre toute valeur supérieure à 2, généralement comprise entre 2 et 14.

15. Procédé selon les revendications 1 ou 2, caractérisé en ce que le précurseur azoté est un aminonitrile représentable par l'une ou l'autre des formules
R₆―NH―[―(CH₂)n―NH―]m―(CH₂)₂―C≡N
ou 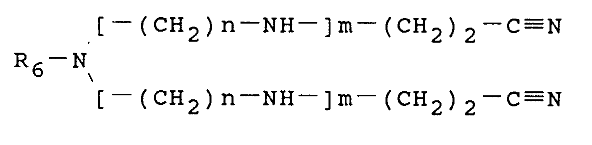 où n est un entier pouvant prendre les valeurs 2 ou 3,
où m peut prendre toute valeur entière ou statistique de O à 3,
où R₆ est H ou CH₃ ou R₁.

## Claims

1. Process for preparing methylated tertiary amines, characterised in that hexamethylenetetramine is reacted with nitrogenous precursors taken from the group consisting of the primary or secondary amines, nitriles and aminonitriles, the reaction being carried out under hydrogen pressure and in the presence of a hydrogenation catalyst, at a temperature not greater than 160°C.

2. Process according to Claim 1, in which the catalyst is a supported nickel hydrogenation catalyst.

3. Process according to Claims 1 or 2, characterised in that the nitrogenous precursor is a primary monoamine R₁NH₂, where R₁ is:
- a linear or branched, saturated or unsaturated hydrocarbon residue R containing from 10 to 22 carbon atoms, or
- a HO-CH₂-CH₂- or HO-CH(CH₃)-CH₂- group, or
- a group R-O-(CH₂)₃-, in which R is a linear or branched, saturated or unsaturated hydrocarbon residue containing from 10 to 20 carbon atoms, or
- a group R-(OCH₂-CH₂)ₚ, R-[OCH(CH₃)-CH₂]ₚ or R-[OCH₂-CH(CH₃)]ₚ, in which R has the same meaning as above and where p is a number which can take any positive statistical value.

4. Process according to Claims 1 or 2, characterised in that the nitrogenous precursor is a secondary monoamine R₁NHR₂,
where R₁ has the same definition as in Claim 3,
where R₂ can be CH₃ or R₁,
it being possible for R₁ and R₂ to be closed in a cyclic amine, it being possible for the ring to secondarily contain another nitrogen or oxygen heteroatom.

5. Process according to Claim 4, characterised in that the nitrogenous precursor is monoethanolamine or diethanolamine.

6. Process according to Claims 1 or 2, characterised in that the nitrogenous precursor is a primary diamine H₂N-(CH₂)ᵣ-NH₂ where r is an integer which can take any value equal to or greater than 2, generally between 2 and 16.

7. Process according to Claims 1 or 2, characterised in that the nitrogenous precursor is a polyamine which can be represented by the general formula 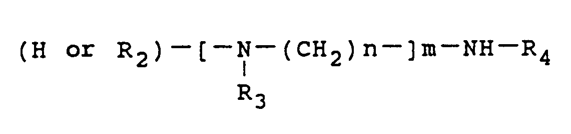 where R₁ has the same definition as in Claim 3,
where R₂ can be CH₃ or R₁,
where R₃ can be H or CH₃ or R₁,
where R₄ is H or CH₃ or R or R₁,
where n is an integer which can take the values 2 or 3,
where m is an integer or any number with a statistical value greater than 1,
it being possible, moreover, for R₂ and R₃ to be closed to form a cyclic amine or even a nitrogenous ring containing another nitrogen or oxygen hetero atom.

8. Process according to Claims 1 or 2, characterised in that the nitrogenous precursor is a fatty polyamine corresponding to one or the other of the formulae
R-NH-[(CH₂)ₙ-NH]ₘ-(CH₂)ₙ-NH₂
or 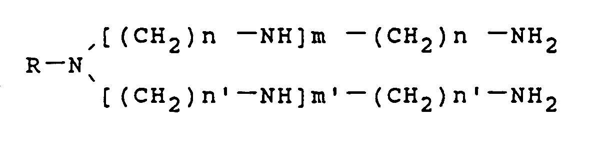 where R is a hydrocarbon chain containing from 10 to 22 carbon atoms;
where n and n' are integers which can take the values 2 or 3;
where m and m' can take any integral or statistical value from 0 to 3.

9. Process according to Claim 8, characterised in that the nitrogenous precursor is a fatty diamine corresponding to the general formula T-NH-(CH₂)₃-NH₂, T being a tallow-based hydrocarbon chain.

10. Process according to Claim 8, characterised in that the nitrogenous precursor is a fatty polyamine containing a tallow chain, of formula RNH-[(CH₂)₃-NH]ₘ-(CH₂)₃-NH₂, where m has a statistical value of approximately 2 to 3.

11. Process according to Claims 1 or 2, characterised in that the nitrogenous precursors are fatty amidoamines or amidopolyamines which can be represented by R-CO-[NH-(CH₂)ₙ-]ₘ-NH-(CO-R or H), where R is a hydrocarbon residue as defined above, where n generally has the value 2 or 3, and m a statistical value from 1 to 6, as well as their derivatives more or less cyclised to imidazolines or tetrahydropyrimidines.

12. Process according to Claims 1 or 2, characterised in that the nitrogenous precursor is a mononitrile which can be represented by the general formula
R₅-C≡N
where R₅ is R or R-O-(CH₂)₂-, R having the definition given above.

13. Process according to Claims 1 or 2, characterised in that the nitrogenous precursor is an aminonitrile which can be represented by the general formula 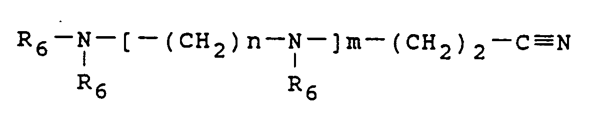 in which
- the R₆ groups can be, independently of each other, H or CH₃ or R₁,
it being possible for two R₆ groups, when they are substituents on the same nitrogen, to additionally constitute a ring with a nitrogen or oxygen hetero atom,
and where
- n is an integer which can take the values 2 or 3,
- m can take any value from 0 to 3.

14. Process according to Claims 1 or 2, characterised in that the nitrogenous precursor is a dinitrile
N≡C-(CH₂)_{q}-C≡N
where q is an integer which can take any value greater than 2, generally of between 2 and 14. 15. Process according to Claims 1 or 2, characterised in that the nitrogenous precursor is an aminonitrile which can be represented by one or the other of the formulae
R₆-NH-[-(CH₂)ₙ-NH-]ₘ-(CH₂)₂-C≡N
or 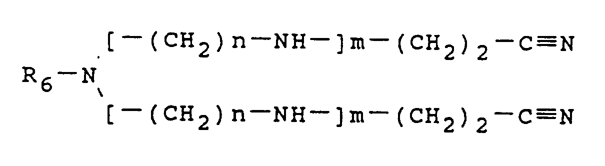 where n is an integer which can take the values 2 or 3,
where m can take any integral or statistical value from 0 to 3,
where R₆ is H or CH₃ or R₁.

## Patentansprüche

1. Verfahren zur Herstellung von methylierten tertiären Aminen, dadurch gekennzeichnet, daß Hexamethylentetramin mit stickstoffhaltigen Vorstufen aus der Gruppe der primären oder sekundären Amine, Nitrile und Aminonitrile umgesetzt wird, wobei die Reaktion unter Wasserstoffdruck und in Gegenwart eines Hydrierungskatalysators bei einer Temperatur von höchstens 160 °C durchgeführt wird.

2. Verfahren nach Anspruch 1, bei dem der Katalysator ein Hydrierungskatalysator aus Nickel auf einem Trägermaterial ist.

3. Verfahren nach einem der Ansprüche 1 oder 2, dadurch gekennzeichnet, daß die stickstoffhaltige Vorstufe ein primäres Monoamin R₁NH₂ ist, wobei R₁
- ein linearer oder verzweigter, gesättigter oder ungesättigter Kohlenwasserstoffrest R mit 10 bis 22 Kohlenstoffatomen ist, oder
- eine HO―CH₂―CH₂― oder eine HO―CH(CH₃)―CH₂-Gruppe ist oder
- eine R―O―(CH₂)₃-Gruppe ist, wobei R ein linearer oder verzweigter, gesättigter oder ungesättigter Kohlenwasserstoffrest mit 10 bis 20 Kohlenstoffatomen ist, oder
- eine R―(OCH₂―CH₂)ₚ-, eine R―[OCH(CH₃)―CH₂]ₚ- oder eine R―[OCH₂―CH(CH₃)]ₚ-Gruppe ist, in der R die gleiche Bedeutung wie oben hat und p eine Zahl ist, die jeden positiven statistischen Wert annehmen kann.

4. Verfahren nach einem der Ansprüche 1 oder 2, dadurch gekennzeichnet, daß die stickstoffhaltige Vorstufe ein sekundäres Monoamin R₁NHR₂ ist, wobei R₁ die gleiche Bedeutung wie in Anspruch 3 hat und R₂CH₃ oder R₁ sein kann, wobei
R₁ und R₂ Teil eines cyclischen Amins sein können, und der Ring zusätzlich ein weiteres Stickstoff- oder Sauerstoff-Heteroatom enthalten kann.

5. Verfahren nach Anspruch 4, dadurch gekennzeichnet, daß die stickstoffhaltige Vorstufe Monoethanolamin oder Diethanolamin ist.

6. Verfahren nach Anspruch 1 oder 2, dadurch gekennzeichnet, daß die stickstoffhaltige Vorstufe ein primäres Diamin H₂N―(CH₂)ᵣ―NH₂ ist, wobei r eine ganze Zahl größer oder gleich 2, im allgemeinen zwischen 2 und 16 ist.

7. Verfahren nach einem der Ansprüche 1 oder 2, dadurch gekennzeichnet, daß die stickstoffhaltige Vorstufe ein Polyamin der allgemeinen Formel 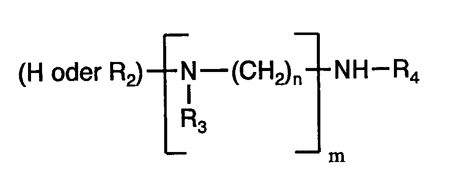 ist, in der
R₁ die gleiche Bedeutung wie in Anspruch 3 hat,
R₂ CH₃ oder R₁ sein kann,
R₃ H oder CH₃ oder R₁ sein kann,
R₄ H, CH₃, R oder R₁ sein kann,
n eine ganze Zahl mit dem Wert 2 oder 3 ist,
m eine ganze oder beliebige Zahl mit einem statistischen Wert von größer 1 ist, wobei
R₂ und R₃ ferner in einem cyclischen Amin oder in einem stickstoffhaltigen Ringsystem mit einem weiteren Stickstoff- oder Sauerstoff-Heteroatom enthalten sein können.

8. Verfahren nach einem der Ansprüche 1 oder 2, dadurch gekennzeichnet, daß die stickstoffhaltige Vorstufe ein Polyfettamin mit einer der beiden folgenden Formeln ist: 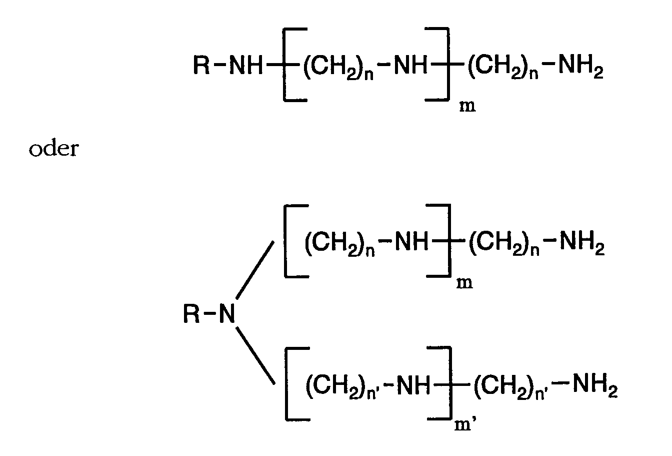 in denen R eine Kohlenwasserstoffkette mit 10 bis 22 Kohlenstoffatomen ist;
n und n' ganze Zahlen mit einem Wert von 2 oder 3 sind; und
m und m' jeden beliebigen ganzzahligen oder statistischen Wert von 0 bis 3 besitzen können.

9. Verfahren nach Anspruch 8, dadurch gekennzeichnet, daß die stickstoffhaltige Vorstufe ein Fettdiamin mit der allgemeinen Formel S―NH―(CH₂)₃―NH₂ ist, wobei S eine Kohlenwasserstoffkette auf Talgbasis ist.

10. Verfahren nach Anspruch 8, dadurch gekennzeichnet, daß die stickstoffhaltige Vorstufe ein Polyfettamin mit einer Kohlenwasserstoffkette auf Talgbasis der Formel 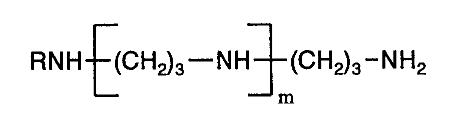 ist, in der m einen statistischen Wert von etwa 2 bis 3 besitzt.

11. Verfahren nach einem der Ansprüche 1 oder 2, dadurch gekennzeichnet, daß die stickstoffhaltigen Vorstufen Fettamidoamine oder Fettamidopolyamine sind, dargestellt durch die Formel 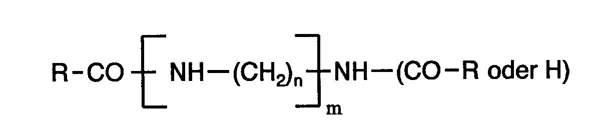 wobei R ein in einem der vorhergehenden Ansprüche definierter Kohlenwasserstoffrest ist und n im allgemeinen 2 oder 3 ist, und m einen statistischen Wert von 1 bis 6 besitzt, sowie deren mehr oder weniger cyclisierte Imidazolin- oder Tetrahydropyrimidinderivate.

12. Verfahren nach einem der Ansprüche 1 oder 2, dadurch gekennzeichnet, daß die stickstoffhaltige Vorstufe ein Mononitril, dargestellt durch die allgemeine Formel
R₅-C≡N
ist, in der R₅ R oder R―O―(CH₂)₂- ist und R die obige Definition hat.

13. Verfahren nach einem der Ansprüche 1 oder 2, dadurch gekennzeichnet, daß die stickstoffhaltige Vorstufe ein Aminonitril, dargestellt durch die allgemeine Formel 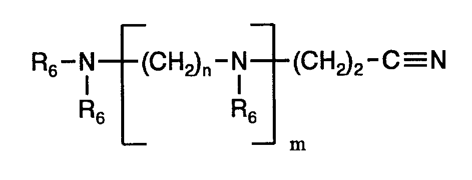 ist, in der
die R₆-Reste unabhängig voneinander H oder CH₃ oder R₁ sein können, zwei R₆-Reste ferner zusammen mit einem Stickstoff- oder Sauerstoff-Heteroatom einen Ring bilden können, wenn sie beide Substituenten an dem gleichen Stickstoffatom sind,
und
- n eine ganze Zahl mit einem Wert von 2 oder 3 ist, und
- m jeden Wert zwischen 0 und 3 annehmen kann.

14. Verfahren nach einem der Ansprüche 1 oder 2, dadurch gekennzeichnet, daß die stickstoffhaltige Vorstufe ein Dinitril
N≡C-(CH₂)_{q}-C≡N
ist, in der q eine ganze Zahl größer 2, im allgemeinen zwischen 2 und 14 ist.

15. Verfahren nach einem der Ansprüche 1 oder 2, dadurch gekennzeichnet, daß die stickstoffhaltige Vorstufe ein Aminonitril, dargestellt durch eine der beiden Formeln: 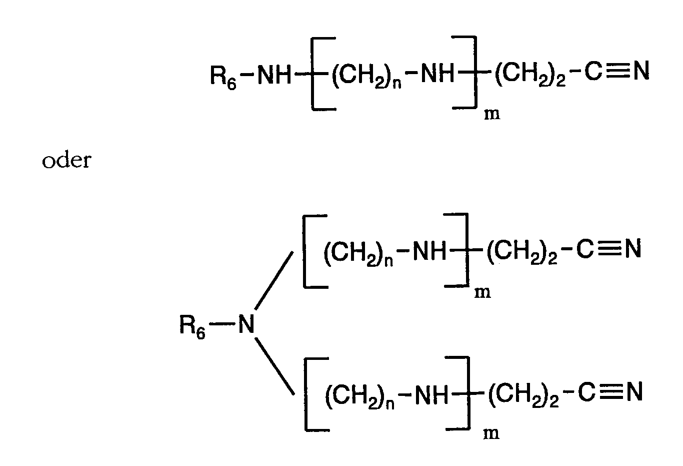 ist, in denen n eine ganze Zahl mit einem Wert von 2 oder 3 ist, und
m jeden ganzen oder statistischen Wert von 0 bis 3 annehmen kann,
und R₆ H oder CH₃ oder R₁ ist.
